(19) Europäisches Patentamt
European Patent Office
Office européen des brevets

(11) Veröffentlichungsnummer: **0 002 690**
**B1**

(12) **EUROPÄISCHE PATENTSCHRIFT**

(21) Anmeldenummer: **78101575.5**

(51) Int. Cl.³: **A 61 K 7/16**

(22) Anmeldetag: **06.12.78**

# (54) Zahnpasta und Verwendung eines synthetischen Alkalialuminiumsilikats als antikorrosiver Zusatz darin

(30) Priorität: **22.12.77 DE 2757280**

(43) Veröffentlichungstag der Anmeldung:
**11.07.79 Patentblatt 79/14**

(45) Bekanntmachung des Hinweises auf die Patenterteilung:
**01.10.80 Patentblatt 80/20**

(84) Benannte Vertragsstaaten:
**BE CH DE FR GB IT NL SE**

(56) Entgegenhaltungen:
**DE - C - 824 285**
**FR - A - 930 740**
**FR - A - 1 047 979**
**FR - A - 2 211 211**
**GB - A - 1 476 063**
**US - A - 2 470 906**
**Chemical Abstracts, Vol. 81, Nr. 18, 4.11.74, Columbus, Ohio, US, Seite 389, Auszug Nr. 111409 g**
**Chemical Abstracts, Vol. 81, Nr. 24, 16.12.74, Columbus, Ohio, US, Seite 358, Auszug Nr. 158 641 s**

(73) Patentinhaber: **Blendax-Werke**
**R. Schneider GmbH & Co.**
**Rheinallee 88**
**D - 6500 Mainz (DE)**

(72) Erfinder: **Harth, Helmut, Dr.**
**Am Gonsenheimer Spiess 63**
**D - 6500 Mainz (DE)**
**Becker, Dieter**
**Messeler Parkstrasse 21**
**D - 6100 Darmstadt-Wixhausen (DE)**

EP 0 002 690 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Zahnpasta und Verwendung eines synthetischen Alkalialuminiumsilikats als antikorrosiver Zusatz darin

Die Erfindung betrifft die Verwendung eines bestimmten synthetischen Alkalialuminiumsilikats vom Zeolith-Typ als antikorrosiver Zusatz in einer Zahnpasta, die gegenüber blanken Aluminiumoberflächen keine Korrosionswirkung aufweist.

Die meisten zum Einsatz in Zahnpasten vorgeschlagenen Poliermittel weisen den Nachteil auf, daß sie auf blanke, das heißt unlackierte Aluminiumflächen eine korrodierende Wirkung ausüben. Dies hat zur Folge, daß die zum Einsatz gelangenden Zahnpastatuben mit einem Innenschutzlack versehen werden müssen, was natürlich die Kosten für das Verpackungsmaterial und damit auch für die fertige Zahnpasta erhöht und darüberhinaus bei eventuellen Schäden in der Innenschutzlackschicht trotzdem zu "Bombagen" der Zahnpastatuben und damit zu Reklamationen führen kann.

Es wurde nun gefunden, daß man eine nicht korrosive Zahnpasta herstellen kann, wenn man dieser Zahnpasta ein Alkalialuminiumsilikat vom Zeolith-Typ in Konzentrationen von 0,05 bis 5 Gew.-%, zusetzt.

Diese Verbindungen sind an sich bereits seit längerem bekannt; sie finden insbesondere als Molekularsiebe, zur Wasserenthärtung und neuerdings auch in Waschmitteln als Ersatzstoffe für die sonst als anorganische Builder eingesetzten Phosphate Anwendung. Es handelt sich dabei um wasserhaltige Gerüstsilikate der Formel $x(M \cdot AlO_2) \cdot ySiO_2 \cdot zH_2O$ wobei M Alkali- oder Ammonium, x eine Zahl zwischen 1 und 64, y eine von x abhängige Zahl mit der Maßgabe, daß sie das ein-bis sechsfache von x und z eine Zahl zwischen 0 und 256 bedeuten.

Die erfindungsgemäß als Poliermittel in Zahnpasten eingesetzten synthetischen Zeolithe lassen sich auf einfache und an sich bekannte Weise aus Aluminiumhydroxid und Alkalisilikaten wie Wasserglas herstellen. Ein im Rahmen der Erfindung besonders geeignetes Produkt ist ein Zeolith A mit der empirischen Formel $Na_{12}(AlO_2)_{12}, (SiO_2)_{12} \cdot 27\ H_2O$. Ein solches Produkt wird von der Firma Degussa unter dem Handelsnamen "Sasil" vertrieben und weist eine mittlere Teilchengröße von etwa 4$\mu$, ein Schüttgewicht von etwa 400 g/l und einen Glühverlust von etwa 20% (1 h bei 800°C) auf. Die zum Einsatz gelangenden Zeolithe sind, wie an sich nicht betont zu werden braucht, in Wasser unlöslich. Ihre durchschnittliche mittlere Teilchengröße liegt vorzugsweise zwischen etwa 1 und etwa 30 Mikron. Eine Übersicht über die Herstellung und die Eigenschaften der erfindungsgemäß zum Einsatz gelangenden Alkalialuminiumsilikate findet sich bei F. Schwochow und L. Puppe, Angewandte Chemie 87 (1975), S. 659 bis 667.

Aus der DE—OS Nr. 2 146 224 sind zwar bereits transparente bzw. transluzente Zahnpasten bekannt, die als Putzkörper ein synthetisches amorphes komplexes Alkali- oder Erdalkalialuminosilikat enthalten, das einen Brechungsindex von etwa 1,44 bis 1,47 aufweist. Die dort beschriebenen Alkalialuminiumsilikate unterscheiden sich jedoch wesentlich von den anmeldungsgemäß zum Einsatz gelangenden Produkten, da sie keine synthetischen Zeolithe darstellen; darüberhinaus ist der Gegenstand dieser DE—OS hinsichtlich der Aufgabenstellung (Herstellung transparenter Zahnpasten) von dem erfindungsgemäßen Vorschlag (Herstellung antikorrosiver Zahnpasten) völlig unterschiedlich. Auch müssen die erfindungsgemäßen Zahnpasten nicht transparent bzw. transluzent sein, der Brechungsindex der erfindungsgemäß eingesetzten Zeolithe liegt im Gegenteil außerhalb des für transparente Zahnpasten erforderlichen Bereiches.

An sich korrosive Poliermittel, die gemeinsam mit dem korrosionsstabilisierenden Zeolith in den erfindungsgemäßen Zahnpasten Einsatz finden können, sind beispielsweise die verschiedenen Calciumphosphate wie Dicalciumorthophosphat in seiner wasserfreien oder hydrathaltigen Form, Calciumpyrophosphat und Tricalciumphosphat, Calcium- oder Magnesiumcarbonat, Aluminiumhydroxid oder unlösliches Alkalimetaphosphat. Der Anteil des Poliermittels liegt üblicherweise zwischen 15 und 60, insbesondere zwischen 20 und 40% Masse.

Es sind bereits zahlreiche Vorschläge unterbreitet worden, Zahnpasten korrosionsverhütende Zusätze einzuverleiben. Als repräsentativ für den einschlägigen Stand der Technik seien hierzu die DE—OSS Nr. 1 953 943 die den Zusatz von Monofluorphosphaten vorschlägt, Nr. 1 953 944, die sich auf die Anwendung von Orthophosphaten bezieht, Nr. 2 509 399, die ein Kieselerdesol, dessen Teilchen negativ geladen sind, offenbart, und Nr. 2 600 709, die oberflächenaktive anionische Phosphatester für diesen Zweck einsetzt, genannt. Die hier beschriebenen Korrosionsstabilisatoren weisen jedoch durchwegs den Nachteil auf, daß sie nur für Aluminiumhydroxid al Poliermittel enthaltende Zahnpasten geeignet sein sollen. Die erfindungsgemäß enthaltenen Korrosionsstabilisatoren sind jedoch zur Stabilisierung aller poliermittelhaltigen Zahnpasten geeignet.

Die Anwendung von Natriumsilikaten als antikorrosive Zusätze in Zahnpasten ist an sich bekannt.

Dabei handelt es sich jedoch, im Gegensatz zu den anmeldungsgemäß zum Einsatz gelangenden Silikaten der allgemeinen Formel $x(M \cdot AlO_2) \cdot ySiO_2 \cdot zH_2O$ vom Zeolith-Typ der vorstehend angegebenen Bedeutung, um einfache Alkalisilikate wie Natriumsilikat, $Na_2SiO_3$.

Diese bekannten korrosionshemmenden Mittel weisen jedoch den großen Nachteil auf, daß sie nur bei pH-Werten von 8,5 und darüber

wirksam sind, d.h., diese Korrosionsschutzmittel sind praktisch nur in alkalischen Zahnpasten, die als Poliermittel Calciumcarbonat enthalten, einzusetzen. Hingegen zeigen die Alkalisilikate die des genannten Typs bei pH-Werten von weniger als 8,5, die meisten Zahnpasten aufweisen, keinerlei korrosionsverhindernde Eigenschaften gegen das in Zahnpastatuben üblicherweise verwendete Aluminium.

Die erfindungsgemäß zu verwendenden korrosionsverhindernden Mittel sind hingegen in ihren korrosionsverhindernden Eigenschaften nicht vom pH-Wert abhängig; sie können in Zahnpasten mit jedem beliebigen Poliermittel zum Einsatz gelangen, was insbesondere im Hinblick auf die bekanntermaßen bei den schwach saure pH-Werte aufweisenden fluorhaltigen kariesprophylaktischen Zahnpasten von besonderer Bedeutung ist. Diese Wirkung der erfindungsgemäß zum Einsatz gelangenden, komplexen Aluminiumsilikate bestimmter Struktur vom Zeolith-Typ war daher besonders überraschend, da der Fachmann von dem ihm bekannten Verhalten der einfachen Alkalisilikate erwarten mußte, daß auch die Zeolithe lediglich im alkalischen Medium wirksam sein würden.

Die GB—PS 1 476 063 erwähnt den Einsatz von Natriumsilikat als antikorrosives Mittel. Dies erscheint in den dort beschriebenen Pasten auch durchaus sinnvoll, da diese auf Grund des verwendeten Poliermittels Calciumcarbonat einen alkalischen pH-Wert aufweisen. Die weiters in dieser Entgegenhaltung beschriebenen "Aluminosilikate" unterscheiden sich von den erfindungsgemäß zum Einsatz gelangenden Zeolithen grundsätzlich durch ihr Verhältnis von Aluminium zu Silicium, das weit außerhalb der Verhältnisse liegt, wie sie bei den erfindungsgemäß eingesetzten Zeolithen anzutreffen sind (vgl. insbesondere S. 1, Z. 38 bis 42 der GB—PS).

Die GB—PS Nr. 332 142 bezieht sich auf Zahnpulver, die Zeolith als Calciumionen-Komplexbildner enthalten. Von einer korrosionsverhindernden Eigenschaft des Zeoliths in Zahnpastatuben ist in dieser Veröffentlichung nicht die Rede. Das einzige Ausführungsbeispiel der GB—PS 332 142 beschreibt den Einsatz von etwa 40% Masse Zeolith in einem Zahnpulver und liegt damit auch weit außerhalb der erfindungsgemäß den Einsatz von Zeolith als korrosionsverhinderndes Mittel definierenden Mengenbereiche. Aufgabenstellung und erst recht Aufgabenlösung der vorliegenden Erfindung lassen sich der GB—PS 332 142 also nicht entnehmen.

Das gleiche gilt hinsichtlich der DE—PS Nr. 378 010, die ebenfalls den Einsatz von Zeolith als Erdalkaliionen-Komplexbildner in Zahnreinigungsmitteln beschreibt.

Die erfindungsgemäßen Zahnpasten enthalten die üblichen Zusatz-und Aufbaustoffe. Als Feuchthaltemittel finden Glycerin, Polyglykole mit niederem Molgewicht oder Zuckeralkohole wie Sorbit, Mannit und Xylit Verwendung.

Zahnpasten enthalten ferner Verdickungsmittel. Als solche eignen sich am besten die Alkalisalze der Carboxymethylcellulose, insbesondere Natriumcarboxymethylcellulose, Hydroxyalkylcellulose, insbesondere Hydroxyäthylcellulose, Pflanzengummen wie Traganth, Gummi arabicum, Caraya-Gummi und Irish Moos, synthetische Polyelektrolyte wie das Natrium-, Kalium- oder Ammoniumsalz der Polyacrylsäure und gegebenenfalls auch anorgansiche Verdickungsmittel, beispielsweise kolloidales Magnesiumaluminiumsilikat.

Der Anteil des Verdickungsmittels beträgt 0,25 bis 5 Gew.-% der Zahnpasta.

Weiterer häufiger Bestandteil von Zahnpasten sind oberflächen aktive Substanzen.

Als solche eignen sich besonders wasserlösliche Salze von höheren Alkylsulfaten, beispielsweise Natriumlaurylsulfat, aliphatische Acylamide gesättigter Monoaminocarbonsäuren, vorzugsweise Natrium-N-lauroylsarcosinat, Taurin-Fettsäureamide, beispielsweise Natrium-N-alkyl-N-myristoyltaurid, Salze von sulfonierten Monoglyceriden höherer Fettsäuren, beispielsweise Natriummonoglyceridsulfonat, Fettsäureester der Isäthionsäure und deren Salze, nichtionische Tenside wie Alkylenoxidkondensate mit Fettalkoholen und ein- oder mehrwertigen Aminen, Zuckerester, beispielsweise Saccharosemonolaurat, Sorbitpolyoxyäthylenstearat, langkettige Aminoxide, beispielsweise Dimethyllaurylaminoxid, ampholytische Tenside, beispielsweise Betaine oder langkettige Alkylaminocarbonsäuren und kationaktive Tenside, beispielsweise quartäre Ammoniumverbindungen wie Cetyltrimethylammoniumbromid.

Der Anteil an oberflächenaktiven Verbindungen in dem erfindungsgemäßen Zahnpflegemittel liegt bei 0 bis 5 Gew.-% der Gesamtzusammensetzung.

Zahnpflegemittel enthalten üblicherweise Aroma- und Geschmackstoffe, Konservierungsmittel etc., derartige Mittel sind an sich bekannt und in zahlreichen Druckschriften beschrieben.

Es ist eine bevorzugte Ausführungsform der Erfindung, Fluorverbindungen in dem erfindungsgemäßen Zahnpflegemittel mitzuverwenden, vorzugsweise in solchen Menge, daß die Konzentration an reinem Fluor im Mittel 0,01 bis 1 Gew.-%, vorzugsweise 0,1 bis 0,5 Gew.-% des Zahnpflegemittels beträgt.

Die erfindungsgemäßen Zahnpflegemittel können weitere, zur Verwendung in solchen Mitteln an sich bekannten Stoffe enthalten, beispielsweise Enzyme wie Proteasen und Carbohydrasen wie Amylase, Dextranase, Lävanase oder $\alpha$-1,3-Glucan-3-glucanohydrolase, Zahnstein entfernende Substanzen wie die für diesen Zweck vorgeschlagenen Phosphonsäuren, beispielsweise Hydroxyäthan-1,1-diphosphonsäure, oder die unter dem Namen "Chlorhexidin", "Fluorhexidin" oder "Alexidin"

bekannten Bisbiguanide 1,6-Di-4'-(chlorphenyldiguanido)hexan, 1,6-Di-4'-(fluorphenyldiguanido)hexan und 1,6-Di-(2-äthylhexyldiguanido)hexan bzw. deren vorzugsweise wasserlösliche Salze.

Eine ausführliche Übersicht über die Herstellung von Zahnpflegemitteln und die dabei zum Einsatz gelangenden Stoffe findet sich in dem Handbuch von M.S. Balsam und E. Sagarin, "Cosmetics — Science and Technology", 2nd Ed., Vol. 1, S. 423 bis 531 (1972). Im folgenden werden einige Beispiele für erfindungsgemäß zusammengesetzte Zahnpflegemittel gegeben:

Beispiel 1

| | |
|---|---|
| Carboxymethylcellulose | 1,20 Gew.-% |
| p-Hydroxybenzoesäure-propylester, Natriumsalz | 0,10 Gew.-% |
| Formalin | 0,10 Gew.-% |
| Sorbit, 70%ig | 12,00 Gew.-% |
| Natriummonofluorphosphat | 0,75 Gew.-% |
| Aroma | 1,00 Gew.-% |
| Saccharinat-Natrium | 0,08 Gew.-% |
| Natriumlaurylsulfat | 1,80 Gew.-% |
| Calciumcarbonat | 35,00 Gew.-% |
| Natriumaluminiumsilikat (Zeolith A, $Na_{12}(AlO_2)_{12}$ $(SiO_2)_{12} \cdot 27H_2O$) | 1,00 Gew.-% |
| Allantoin | 0,20 Gew.-% |
| pyrogene Kieselsäure | 2,00 Gew.-% |
| Wasser | 44,77 Gew.-% |

Beispiel 2

| | |
|---|---|
| Natriumalginat | 1,00 Gew.-% |
| p-Hydroxybenzoesäure-methylester, Natriumsalz | 0,10 Gew.-% |
| Glycerin | 25,00 Gew.-% |
| Kaliummonofluorphosphat | 0,75 Gew.-% |
| Aroma | 1,00 Gew.-% |
| Bromchlorophen | 0,05 Gew.-% |
| Saccharin-Natrium | 0,06 Gew.-% |
| Farbstoff | 0,02 Gew.-% |
| Dicalciumphosphat-dihydrat | 45,00 Gew.-% |

Beispiel 2 (fortsetzung)

| | |
|---|---|
| Dicalciumphosphat, wasserfrei | 7,00 Gew.-% |
| Natriumaluminiumsilikat (Zeolith A, $Na_{12}(AlO_2)_{12}$ $(SiO_2)_{12} \cdot 27H_2O$) | 0,50 Gew.-% |
| Natriumlaurylsulfat | 1,60 Gew.-% |
| Wasser | 17,92 Gew.-% |

Beispiel 3

| | |
|---|---|
| Carboxymethylcellulose | 1,00 Gew.-% |
| Formalin | 0,05 Gew.-% |
| p-Hydroxybenzoesäure-methylester | 0,07 Gew.-% |
| 1,2-Propylenglykol | 3,00 Gew.-% |
| Glycerin | 17,00 Gew.-% |
| Aroma | 1,20 Gew.-% |
| Dicalciumphosphat | 40,00 Gew.-% |
| Natriumaluminiumsilikat ($Na_2O \cdot Al_2O_3 \cdot 2SiO_2 \cdot 4{,}5H_2O$) | 2,00 Gew.-% |
| Saccharinat-Natrium | 0,12 Gew.-% |
| Natriumcyclamat | 0,02 Gew.-% |
| Natriummonofluorphosphat | 0,75 Gew.-% |
| Kräuterextrakt | 1,50 Gew.-% |
| Farbstoff | 0,01 Gew.-% |
| Natriumlaurylsulfat | 2,00 Gew.-% |
| Wasser | 31,28 Gew.-% |

Beispiel 4

| | |
|---|---|
| Carboxymethylcellulose | 1,10 Gew.-% |
| Benzoesäure | 0,20 Gew.-% |
| p-Hydroxybenzoesäure-propylester | 0,05 Gew.-% |
| Glycerin | 20,00 Gew.-% |
| 1,2-Propylenglykol | 4,00 Gew.-% |
| Tetracalciumpyrophosphat | 45,00 Gew.-% |
| Natriumaluminiumsilikat (Zeolith A, $Na_{12}(AlO_2)_{12}$ $(SiO_2)_{12} \cdot 27H_2O$) | 4,00 Gew.-% |

Beispiel 4 (fortsetzung)

| | |
|---|---|
| Natriummonofluorphosphat | 0,75 Gew.-% |
| Natriumlauroylsarcosinat | 1,80 Gew.-% |
| Aroma | 1,00 Gew.-% |
| Saccharin-Natrium | 0,09 Gew.-% |
| Wasser | 22,01 Gew.-% |

Das Natriumaluminiumsilikat kann in den beispielhaft angegebenen Rezepturen jeweils ganz oder teilweise durch die entsprechende Lithium- oder Kaliumverbindung ersetzt werden. Die Teilchengrößenverteilung der eingesetzten Zeolithe liegt dabei vorzugsweise so, daß mindestens 97% < 15$\mu$m, mindestens 95% < 10 $\mu$m und etwa 40% < 5 $\mu$m sind.

## Patentansprüche

1. Verwendung von 0,05 bis 5,0 Gew.-% der Gesamtzusammensetzung eines synthetischen Alkalialuminiumsilikats vom Zeolith-Typ der allgemeinen Formel $x(M \cdot AlO_2) \cdot ySiO_2 \cdot zH_2O$, wobei M Alkali- oder Ammonium, x eine Zahl zwischen 1 und 64, y eine von x abhängige Zahl mit der Maßgabe, daß sie das ein- bis sechsfache von x und z eine Zahl zwischen 0 und 256 bedeutet, entsprechen, als antikorrosiver Zusatz in einer Zahnpasta auf Basis einer an sich blanke Aluminiumtuben korrodierenden Grundlage.

2. Zahnpasta auf Basis einer an sich blanke Aluminiumflächen korrodierenden Grundlage, gekennzeichnet durch einen Gehalt an 0,05 bis 5,0 Gew.-% der Gesamtzusammensetzung Natriumaluminiumsilikat entsprechend der empirischen Formel

$$Na_{12}(AlO_2)_{12} \cdot (SiO_2)_{12} \cdot 27H_2O$$

als antikorrosiver Zusatz.

## Claims

1. Use of 0,05 to 5,0% by weight (calculated on the entire composition) of a synthetical alkali aluminium silicate of zeolite-type of the general formula $x(M \cdot AlO_2) \cdot ySiO_z \cdot zH_2O$, wherein M is alkali or ammonium, x is a number from 1 to 64, y is a number dependent on x with the determination of y being one-fold to six-fold of x, and z is a number from 0 to 256, as anti-corrosive admixture in a toothpaste composition corroding blank aluminium tubes.

2. Toothpaste composition on a base corroding blank aluminium tubes *per se*, characterized by a contents of 0,05 to 5,0% by weight (calculated on the entire composition) of sodium aluminium silicate of the empirical formula $Na_{12}(AlO_2)_{12} \cdot (SiO_2)_{12} \cdot 27H_2O$ as anti-corrosive admixture.

## Revendications

1. Utilisation de 0,05 à 5,0% du poids de la composition totale d'un silicate d'aluminium alcalin synthétique du type zéolithe de la formule générale $x(M \cdot AlO_2) \cdot ySiO_2 \cdot zH_2O$, dont M signifie alcali ou ammonium, x un nombre entre 1 et 64, y un nombre dépendant de x avec la mesure qu'il signifie le simple jusqu'au sextuple de x et dont z signifie un nombre entre 0 et 256, comme addition anticorrosive dans un dentifrice sur base corrosive aux tubes d'aluminium non vernis.

2. Dentifrice sur base corrosive aux tubes d'aluminium non vernis, caractérisé par le fait qu'il contient 0,05 à 5,0% du poids de la composition totale d'un silicate d'aluminium alcalin selon la formule empirique $Na_{12}(AlO_2) \cdot (SiO_2)_{12} \cdot 27H_2O$ comme addition anticorrosive.